# EUROPEAN PATENT SPECIFICATION

(11) **EP 4 346 965 B1**
(45) Date of publication and mention of the grant of the patent: **26.02.2025**
(21) Application number: 22731288.1
(22) Date of filing: 01.06.2022
(51) Int. Cl.: A61M 16/08, A61M 16/10

(54) **A BREATHING ASSEMBLY**
BEATMUNGSANORDNUNG
ENSEMBLE RESPIRATOIRE

(30) Priority: 04.06.2021 GB 202108038; 14.01.2022 GB 202200453
(43) Date of publication of application: 10.04.2024
(73) Proprietor: Flexicare (Group) Limited, Mountain Ash, Mid Glamorgan CF45 4ER (GB)
(72) Inventor: THOMAS, Tudor, Mountain Ash Mid Glamorgan CF45 4ER (GB); REES-WHIPPEY, Daniel, Mountain Ash Mid Glamorgan CF45 4ER (GB); POORMAND, Ghassem, Mountain Ash Mid Glamorgan CF45 4ER (GB)
(74) Representative: Mewburn Ellis LLP
(86) International application number: PCT/GB2022/051398
(87) International publication number: WO 2022/254214

(56) References cited:
- WO-A1-2011/077250
- WO-A1-2020/094741
- US-A- 4 463 755
- US-A- 4 967 744
- US-A1- 2004 118 401
- US-A1- 2009 025 724
- US-A1- 2018 110 955
- US-A1- 2018 280 650

## Description

### FIELD

The present teachings relate to a breathing assembly.

### BACKGROUND

Patients with compromised ability to breathe autonomously can have their respiration supported through a breathing system, i.e. through mechanical ventilation. Mechanical ventilation utilises externally generated positive air pressure to induce a pressure gradient to the lungs, leading to a flow in of air. This pressure gradient is varied cyclically, in order to mimic the normal respiration of a patient. With mechanical ventilation, the patient must be connected to the source of the pressure change (e.g. a ventilator) through a breathing system.

In such assisted/support breathing, particularly in medical applications, the breathing system needs to be capable of supplying gases, known as inspiratory gases, to a patient at suitable temperature and humidity levels. In known breathing systems, the high humidity of the conveyed gases can result in the build-up of condensation within the conduits.

Some prior art breathing systems are known from the documents US 2004/118401 A1, WO 2011/077250 A1, WO 2020/094741 A1, US 2018/280650 A1, US 2009/025724 A1 and US 4 967 744 A.

The present teachings seek to overcome or at least mitigate one or more problems associated with the prior art.

### SUMMARY OF THE INVENTION

The present invention provides a breathing assembly for supplying gases to a patient as defined in claim 1. Further preferred embodiments of the present invention are defined in the dependent claims.

### SUMMARY OF THE DISCLOSURE

Reference will now be made to various exemplary embodiments or aspects of the present disclosure. Embodiments or aspects disclosed herein which do not fall under the scope of the appended claims do not form part of the present invention, but are useful for understanding (the principles of) the invention.

The scope of the present invention is defined by the appended claims.

According to a first aspect, there is provided a breathing assembly for supplying gases to a patient, the breathing assembly comprising: a patient end connector configured to be connectable to an airway device; a chamber end connector configured to be connectable to a humidification chamber and comprising an inspiratory inlet for receiving humidified inspiratory gases from the humidification chamber; and a coaxial conduit assembly extending between the patient end connector and the ventilator end connector and comprising inner and outer conduits defined by inner and outer conduit wall, the inner conduit wall arranged within the outer conduit wall so as to define a first flow passage along the coaxial conduit assembly within the inner conduit wall and a second flow passage along the coaxial conduit assembly between the inner conduit wall and outer conduit wall, wherein the breathing assembly is configured such that inspiratory gases are conveyed along the first flow passage and expiratory gases are conveyed along the second flow passage, in use, and wherein the outer conduit wall is at least partially formed from a water permeable material configured to allow water to flow therethrough.

It will be appreciated that the airway device may be an infraglottic airway (e.g. endotracheal tube, endobronchial tube, tracheostomy tube), or a supraglottic airway device (e.g. oropharyngeal airway, nasopharyngeal airway, and laryngeal mask airway).

In this arrangement, the second flow passage provides a thermal barrier between the inspiratory gas in the first flow passage and the ambient air, which facilitates maintaining the inspiratory gas at the required temperature.

Providing an outer conduit that is formed from a water permeable material that allows water to flow therethrough has been found to reduce the build-up of water in the second flow passage, and can also reduce the build-up of water in the ventilator.

The water permeable material may be configured to allow flow of liquid water therethrough and configured to restrict flow of respiratory gases therethrough.

The water permeable material may be configured to restrict the flow of liquid water therethrough.

Providing an outer conduit that is formed from a water permeable material that allows water to flow therethrough and that allows, but restricts, the passage of liquid water and/or gases therethrough has been found to reduce the build-up of water and water vapour in the second flow passage.

Substantially all, e.g. an entirety, of the outer conduit wall may be formed from the water permeable material.

This has been found to enhance the breathability of the outer conduit, thereby further reducing the build-up of water and water vapour in the second flow passage.

The outer conduit wall may be self-supporting.

This removes the need for additional structural members to be mounted to the outer conduit to provide the necessary structural stiffness/rigidity.

The outer conduit wall may comprise a reinforcing arrangement.

This provides the outer conduit with improved structural stiffness/rigidity.

The reinforcing arrangement may comprise a corrugated region of the outer conduit wall, optionally wherein the corrugated region extends over an entirety of the elongate length of the outer conduit wall.

This removes the need for additional structural members to be mounted to the outer conduit to provide the necessary structural stiffness/rigidity.

The outer conduit may be configured to support the inner conduit.

The coaxial conduit assembly may comprise a spacing arrangement between the inner conduit wall and the outer conduit wall.

The spacing arrangement may comprise at least one spacing assembly provided along the elongate length of the coaxial conduit assembly.

Each spacing assembly may comprise a plurality of spacing members positioned between the inner conduit wall and the outer conduit wall.

The plurality of spacing members may be equally spaced around the inner conduit wall.

The spacing members may project inwardly (i.e. radially inwardly) from the outer conduit wall.

The outer conduit may define an outer conduit wall having a thickness in the range 0.2mm to 1.0mm, optionally in the range 0.3mm to 0.9mm.

Providing an outer conduit wall in this range has been found to provide sufficient stiffness/rigidity of the outer conduit whilst providing a suitably water permeable outer conduit wall.

The water permeable material may be formed from an amphiphilic material.

This has been found to enhance the permeability of the outer conduit to water vapour and liquid water, thereby reducing the build-up of water and water vapour in the second flow passage.

The water permeable material may be formed from a hydrophobic and hydrophilic block co-polymer.

The water permeable material may be formed from a hydrophobic and hydrophilic poly (ethylene oxide), poly(butylene terephthalate) (PBT) based block co-polymer.

This has been found to enhance the permeability of the outer conduit to water vapour and liquid water, thereby reducing the build-up of water and water vapour in the second flow passage.

The outer conduit wall may be configured to absorb water vapour and liquid water.

This has been found to further enhance the permeability of the outer conduit to water vapour and liquid water, thereby further reducing the build-up of water and water vapour in the second flow passage.

The inner conduit wall may be insufficiently strong to be self-supporting.

The inner conduit wall may be formed from a thermoplastic elastomer.

The inner conduit wall may be configured to prevent water and water vapour from flowing therethrough.

The inner conduit wall may be configured to prevent gases from flowing therethrough.

This arrangement enables the humidity of the inspiratory gases to be actively, rather than passively, controlled by the breathing assembly.

The inner conduit may comprise a reinforcing arrangement.

Incorporating a reinforcing arrangement enables the thickness of the wall of the inner conduit to be reduced, thus reducing the amount of material used in the inner conduit.

The reinforcing arrangement may comprise a reinforcing rib extending around, e.g. helically around, the inner conduit wall.

This has been found to further improve reinforcement of the inner conduit.

The breathing assembly may comprise a heating arrangement configured and arranged to heat inspiratory gases flowing along the first flow passage, wherein the heating arrangement comprises a first heating member embedded within an inner conduit wall of the inner conduit.

This has been found to improve the packing of the breathing assembly. By arranging the first heating element within the wall of the inner conduit wall, the obstruction to flow of the inspiratory gases is reduced.

The heating arrangement may be configured such that the inspiratory gases maintain an absolute humidity of at least 33 mg/l.

The first heating member may extend around, e.g. spiral around, the inner conduit wall of the inner conduit.

Arranging the first heating element as a spiral around the wall of the inner conduit has been found to provide more uniform heating of the inspiratory gases flowing within the inner conduit.

The first heating member may be configured and arranged to heat inspiratory gases flowing along the first flow passage and to heat expiratory gases flowing along the second flow passage.

Arranging the first heating element so as to heat both inspiratory and expiratory gases can reduce or remove the need for a separate heating arrangement for the expiratory gases.

The first heating member may comprise a heating power in the range 25 to 40 Watts, optionally in the range 27 to 35 Watts, optionally in the range 29 to 33 Watts, for example approximately 31 Watts.

The first heating member may be configured to heat the inspiratory gases to a temperature in the range 36°C to 41°C, for example in the range 37°C to 40°C.

The inner conduit wall may comprise a reinforcing rib extending therearound, and wherein the first heating member is embedded within the reinforcing rib.

Arranging the first heating element within the reinforcing rib has been found to improve the packing of the breathing assembly.

The breathing assembly may comprise a catheter mount connected to the patient end connector.

The breathing assembly may comprise a second temperature sensor at or near the patient end connector.

The second temperature sensor may be integrally formed with the patient end connector. Alternately, the second temperature sensor may be separate from, and connectable to, the patient end connector (i.e. the second temperature sensor may be reusable).

The inner conduit wall may comprise a reinforcing rib extending therearound, and wherein one or more electrical wires connected to the second temperature sensor are embedded within the reinforcing rib.

Arranging the electrical wires within the reinforcing rib has been found to improve the packing of the breathing assembly.

The breathing assembly may comprise a first temperature sensor at or near the ventilator end connector.

The first temperature sensor may be integrally formed with the ventilator end connector. Alternately, the first temperature sensor may be separate from, and connectable to, the ventilator end connector (i.e. the first temperature sensor may be reusable).

The heating arrangement may comprise a second heating member configured to heat gases flowing along the second flow passage, wherein the second heating element is either positioned in the second flow passage or is embedded within a wall of the outer conduit.

The second heating member may be configured to heat the outer conduit wall to a predetermined temperature range or a predetermined temperature.

This enables the second heating member to heat the outer conduit wall to a temperature or temperature range so as to maximise its permeability to water.

The second heating member may comprise a heating power in the range 5 to 40 Watts.

The breathing assembly may comprise an expiratory port for conveying expiratory gasses out of the breathing assembly, wherein the expiratory port is provided at or proximate to the patient end connector

The breathing assembly may comprise a flow valve at or near the interface between the coaxial conduit assembly and the patient end connector, the flow valve configured to allow flow of inspiratory gases from the first flow to passage into the patient end connector and to direct expiratory gases flowing from the patient end connector to the coaxial conduit assembly into the second flow passage.

According to a second aspect, there is provided a breathing assembly for supplying gases to a patient, the breathing assembly comprising: a patient end connector configured to be connectable to an air way device; a ventilator end connector configured to be connectable to a humidification chamber and comprising an inspiratory inlet for receiving humidified inspiratory gases from the humidification chamber; a coaxial conduit assembly extending between the patient end connector and the ventilator end connector and comprising inner and outer conduits defined by inner and outer conduit walls, the inner conduit wall arranged within the outer conduit wall so as to define a first flow passage along the coaxial conduit assembly within the inner conduit wall and a second flow passage along the coaxial conduit assembly between the inner conduit wall and outer conduit wall, wherein the first flow passage and second flow passage each convey one of inspiratory gases or expiratory gases, in use; and a heating arrangement for heating gases flowing along the coaxial conduit assembly, wherein the heating arrangement comprises a first heating member embedded within the inner conduit wall, and wherein the first heating member is configured and arranged to heat gases flowing along the first flow passage and the second flow passage.

This has been found to improve the packing of the breathing assembly. By arranging the first heating element within the wall (i.e. on the outer surface of the wall) of the inner conduit, the obstruction to flow of the inspiratory gases is reduced.

Arranging the first heating element so as to heat both inspiratory and expiratory gases can reduce the heat that needs to be supplied by a dedicated expiratory gas heating arrangement.

It will be appreciated that the airway device may be an infraglottic airway (e.g. endotracheal tube, endobronchial tube, tracheostomy tube), or a supraglottic airway device (e.g. oropharyngeal airway, nasopharyngeal airway, and laryngeal mask airway).

The heating arrangement may be configured such that the inspiratory gases maintain an absolute humidity of at least 33 mg/l.

The first heating member may comprise a heating power in the range 25 to 40 Watts, optionally in the range 27 to 35 Watts, optionally in the range 29 to 33 Watts, for example approximately 31 Watts.

The first heating member may extend around the inner conduit wall.

This has been found to improve the uniformity of heating of the gases flowing within the coaxial conduit assembly.

The first heating member may spiral around the inner conduit wall.

Arranging the first heating element as a spiral around the wall of the inner conduit has been found to provide more uniform heating of the inspiratory gases flowing within the inner conduit.

The inner conduit wall may comprise a reinforcing rib extending therearound, and wherein the first heating member is embedded within the reinforcing rib.

Arranging the first heating element within the reinforcing rib has been found to improve the packing of the breathing assembly.

The first heating member may comprise two spaced apart heating elements, e.g. wires, embedded within the reinforcing rib.

This has been found improve the uniformity of heating of the gases flowing within the coaxial conduit assembly

The provision of two heating elements enables the feed and return current path to be contained within the same assembly.

The heating arrangement may comprise a second heating member configured and arranged to heat gases flowing along the second flow passage, wherein the second heating member is either positioned in the second flow passage or is embedded within the outer conduit wall.

This arrangement helps to further heat expiratory gases to reduce the build-up of water and water vapour in the second flow passage or to heat inspiratory gases to ensure that are provided to the patient at the correct temperature.

Provision of a dedicated heater for the second flow passage enables the gases within the inner and outer flow passages to be heated to separate temperatures.

The breathing assembly may be configured such that inspiratory gases are conveyed along the first flow passage and expiratory gases are conveyed along the second flow passage, in use.

In this arrangement, the second flow passage provides a thermal barrier between the inspiratory gas in the first flow passage and the ambient air, which facilitates maintaining the inspiratory gas at the required temperature.

The first heating member may be configured to heat the inspiratory gases to a temperature in the range 36°C to 41°C, for example in the range 37°C to 40°C.

The outer conduit wall may be at least partially formed from a water permeable material configured to allow water to flow therethrough and configured to restrict flow of respiratory gases therethrough.

The second heating member may be configured to heat the outer conduit wall to a predetermined temperature range or a predetermined temperature.

This enables the second heating member to heat the outer conduit wall to a temperature or temperature range so as to maximise its permeability to water.

The second heating member may comprise a heating power in the range 5 to 40 Watts.

The water permeable material may be configured to restrict the flow of liquid water therethrough.

Providing an outer conduit that is formed from a water permeable material that allows water to flow therethrough and that allows, but restricts, the passage of liquid water therethrough has been found to reduce the build-up of water and water vapour in the second flow passage.

An entirety of the outer conduit wall may be formed from the water permeable material.

This has been found to enhance the breathability of the outer conduit, thereby further reducing the build-up of water and water vapour in the second flow passage.

The outer conduit wall may be self-supporting.

This removes the need for additional structural members to be mounted to the outer conduit to provide the necessary structural stiffness/rigidity.

The outer conduit wall may be configured to support the inner conduit wall.

The coaxial conduit assembly may comprise a spacing arrangement between the inner conduit wall and the outer conduit wall.

The spacing arrangement may comprise at least one spacing assembly provided along the elongate length of the coaxial conduit assembly.

Each spacing assembly may comprise a plurality of spacing members positioned between the inner conduit wall and the outer conduit wall.

The plurality of spacing members may be equally spaced around the inner conduit wall.

The spacing members may project inwardly (i.e. radially inwardly) from the outer conduit wall.

The outer conduit may comprise a reinforcing arrangement.

This provides the outer conduit with improved structural stiffness/rigidity.

The reinforcing arrangement may comprise a corrugated region of the outer conduit wall.

The corrugated region may extend over an entirety of the elongate length of the outer conduit wall.

This removes the need for additional structural members to be mounted to the outer conduit to provide the necessary structural stiffness/rigidity.

The outer conduit may define an outer conduit wall having a thickness in the range 0.2mm to 1.0mm, optionally in the range 0.3mm to 0.9mm.

Providing an outer conduit wall in this range has been found to provide sufficient strength to the outer conduit whilst providing a suitably water permeable outer conduit wall.

The inner conduit wall may be insufficiently strong to be self-supporting.

The inner conduit wall may be formed from a thermoplastic elastomer.

The inner conduit wall may be configured to prevent water and water vapour from flowing therethrough.

The inner conduit may be configured to prevent liquid water and air/gases from flowing therethrough.

This arrangement enables the humidity of the inspiratory gases to be actively, rather than passively, controlled by the breathing assembly.

The inner conduit wall may comprise a reinforcing arrangement.

Incorporating a reinforcing arrangement enables the thickness of the wall of the inner conduit to be reduced, thus reducing the amount of material used in the inner conduit.

The reinforcing arrangement may comprise a reinforcing rib extending around, e.g. helically around, the inner conduit wall.

This has been found to further improve reinforcement of the inner conduit.

The breathing assembly may comprise a flow valve at or near the interface between the coaxial conduit assembly and the patient end connector, the flow valve configured to allow flow of inspiratory gases from the first flow to passage into the patient end connector and to direct expiratory gases flowing from the patient end connector to the coaxial conduit assembly into the second flow passage.

The breathing assembly may comprise a catheter mount connected to the patient end connector.

The breathing assembly may comprise a second temperature sensor at or near the patient end connector.

The second temperature sensor may be integrally formed with the patient end connector. Alternately, the second temperature sensor may be separate from, and connectable to, the patient end connector (i.e. the second temperature sensor may be reusable).

This has been found to improve the packing of the breathing assembly.

The inner conduit wall may comprise a reinforcing rib extending therearound, and wherein one or more electrical wires connected to the second temperature sensor are embedded within the reinforcing rib.

Arranging the electrical wires within the reinforcing rib has been found to improve the packing of the breathing assembly.

The breathing assembly may comprise a first temperature sensor at or near the ventilator end connector.

The first temperature sensor may be integrally formed with the ventilator end connector. Alternately, the first temperature sensor may be separate from, and connectable to, the ventilator end connector (i.e. the first temperature sensor may be reusable).

This has been found to improve the packing of the breathing assembly.

The heating arrangement may comprise a second heating member configured to heat gases flowing along the second flow passage, wherein the second heating element is either positioned in the second flow passage or is embedded within a wall of the outer conduit.

The breathing assembly may comprise an expiratory port for conveying expiratory gasses out of the breathing assembly, wherein the expiratory port is provided at or proximate to the patient end connector.

### BRIEF DESCRIPTION OF THE DRAWINGS

Embodiments will now be described with reference to the accompanying drawings, in which:
Figure 1 is an isometric view of a part of a breathing assembly according to an embodiment;
Figure 2 is a cross-sectional side view of the breathing assembly of Figure 1;
Figure 3 is a cross-sectional partial view of the breathing assembly of Figure 1;
Figure 4 is a cross-sectional partial view of the inner and outer conduits of the breathing assembly of Figure 1;
Figure 5 is a partial cross-sectional partial view of the outer conduit of the breathing assembly of Figure 1;
Figures 6A and 6B are a side view and a cross-sectional side view of an inner conduit of the breathing assembly of Figure 1.

### DETAILED DESCRIPTION OF EMBODIMENT(S)

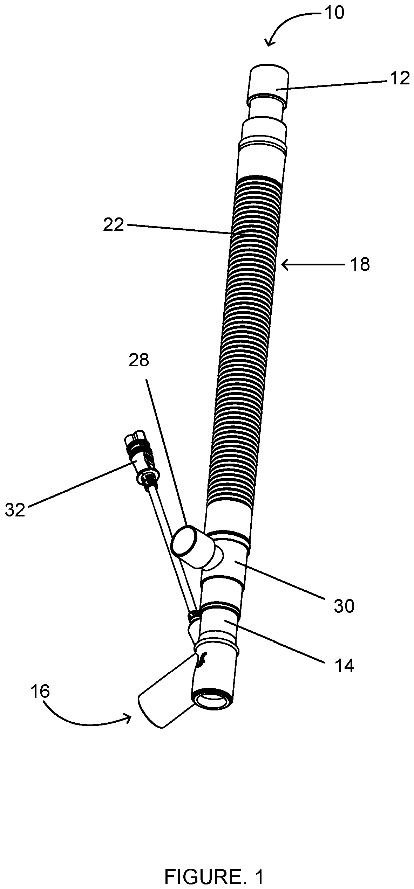

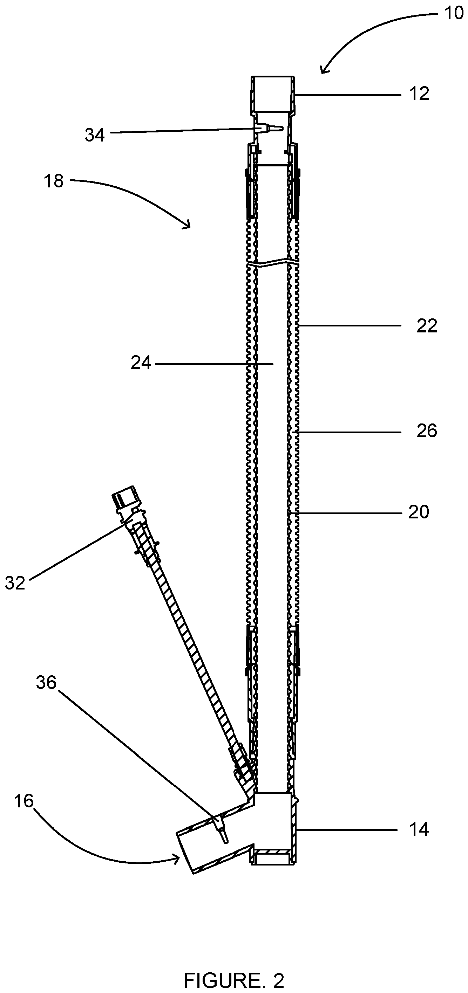

Referring firstly to Figures 1 and 2, a breathing assembly for supplying gases to a patient (not shown) is illustrated and is indicated generally at 10.

The breathing assembly 10 includes a patient end connector 12. Although not illustrated, the breathing assembly 10 may include a catheter mount connected to the patient end connector 12. The patient end connector 12 is configured to be connectable to an air way device. The patient end connector 12 may be configured to be connectable to a catheter mount. The catheter mount, when fitted, transports both inspiratory gases to an airway device and expiratory gases from an airway device. The patient end connector 12 may be configured to be connectable directly to airway devices such an infraglottic airway device (e.g. endotracheal tube, endobronchial tube, tracheostomy tube) or a supraglottic airway device (e.g. oropharyngeal airway, nasopharyngeal airway, and laryngeal mask airway), or any other suitable airway device.

The breathing assembly 10 includes a chamber end connector 14. Although not illustrated, the breathing assembly 10 may be connected to a humidification chamber connected to the chamber end connector 14. Put another way, the chamber end connector 14 is configured to be connectable to a humidification chamber. The chamber end connector 14 includes an inspiratory inlet 16 for receiving inspiratory gases from the humidification chamber.

The breathing assembly 10 includes a coaxial conduit assembly 18. The coaxial conduit assembly 18 extends between the patient end connector 12 and the chamber end connector 14. The coaxial conduit assembly 18 includes an inner conduit 20 and an outer conduit 22. The inner conduit 20 is defined by an inner conduit wall. The outer conduit 22 is defined by an outer conduit wall. The inner conduit 20 is arranged within the outer conduit 22 so as to define a first flow passage 24 along the coaxial conduit assembly 18 within the inner conduit 20 and a second flow passage 26 along the coaxial conduit assembly 18 between the inner conduit 20 and outer conduit 22.

The breathing assembly 10 includes an expiratory port 28. The expiratory port 28 is provided for conveying expiratory gasses out of the breathing assembly 10. In the illustrated arrangement, the expiratory port 28 is provided on a collar 30 interposed between the chamber end connector 14 and the coaxial conduit assembly 18. In alternative arrangements, the expiratory port 28 may be provided on the chamber end connector 14 or may be provided on a region of the coaxial conduit assembly 18 adjacent to, i.e. close to, the chamber end connector 14. Put another way, the expiratory port 28 may be at or proximate to the chamber end connector 14.

The breathing assembly 10 includes an electrical connector 32 for connecting to an electrical source of power. The electrical connector 32 connects the breathing assembly 10 to a control system (not shown). As will be discussed in more detail below, the electrical connector 32 is connected to various components of the breathing assembly 10 such that the components of the breathing assembly 10 can be connected to the control system and/or to provide electrical power to the components of the breathing assembly 10.

Although not illustrated, the breathing assembly 10 may include a flow valve. The flow valve is provided to allow flow of inspiratory gases from the coaxial conduit assembly 18 into the patient end connector 12 and to direct expiratory gases flowing from the patient end connector 12 to the coaxial conduit assembly 18 into the desired first or second flow passage 24, 26. Typically this flow valve is provided at the ventilator (not shown) so as to reduce complexity of the breathing assembly. Alternatively, the flow valve may be provided at or near the interface between the coaxial conduit assembly 18 and the patient end connector 12.

In the illustrated arrangement, the breathing assembly 10 is configured such that inspiratory gases are conveyed along the first flow passage 24 and expiratory gases are conveyed along the second flow passage 26, in use. In this arrangement, the second flow passage 26 (i.e. the outer flow passage) provides a thermal barrier between the inspiratory gas in the first flow passage 24 and the ambient air, which facilitates maintaining the inspiratory gas at the required temperature (e.g. around 37 °C). It will be appreciated, however, that in alternative arrangements, the breathing assembly 10 may be configured such that inspiratory gases are conveyed along the second flow passage 26 and expiratory gases are conveyed along the first flow passage 24, in use.

The breathing assembly 10 includes a first temperature sensor 36. The first temperature sensor 36 is a temperature sensor. The first temperature sensor 36 is positioned at or near the chamber end connector 14. In the illustrated embodiment, the first temperature sensor 36 is integrally formed with the chamber end connector 14. In alternative arrangements, however, it will be appreciated that the first temperature sensor 36 may be removable, e.g. disposable. The first temperature sensor 36 is connected to the electrical connector 32. Put another way, the first temperature sensor 36 is connectable to a control system (not shown).

The breathing assembly 10 includes a second temperature sensor 34. The second temperature sensor 34 is a temperature sensor. The second temperature sensor 34 is positioned at or near the patient end connector 12. In the illustrated embodiment, the second temperature sensor 34 is integrally formed with the patient end connector 12. In alternative arrangements, however, it will be appreciated that the second temperature sensor 34 may be removable, e.g. disposable. The second temperature sensor 34 is connected to the electrical connector 32. Put another way, the second temperature sensor 34 is connectable to a control system (not shown) and/or an electrical source of power via the electrical connector 32. The second temperature sensor 34 is connected to the electrical connector 32 via one or more wires (not shown) extending along the coaxial conduit assembly 18, for example embedded within a wall of the inner conduit 20 or outer conduit 22.

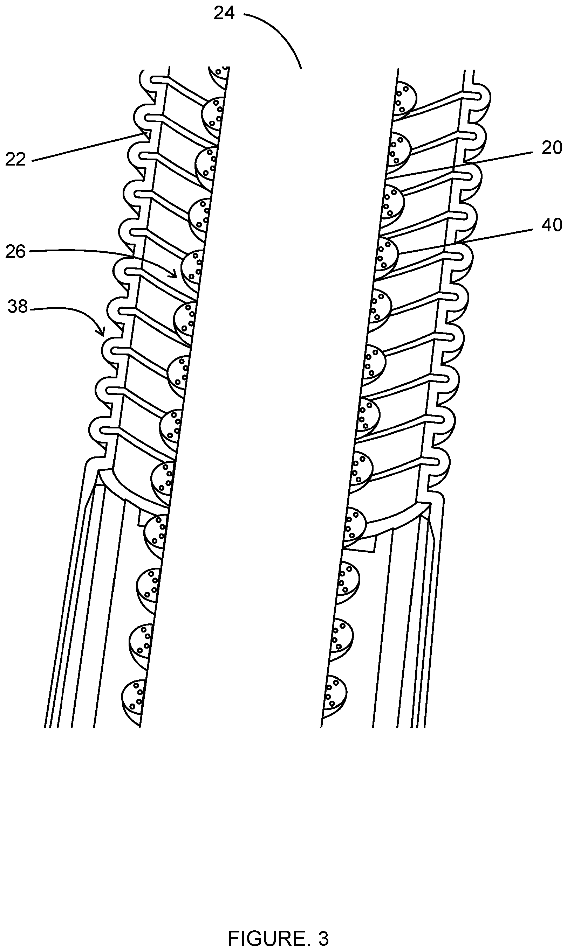

Referring now to Figure 3, the coaxial conduit assembly 18 includes inner and outer conduits 20, 22. The outer conduit wall 22 is at least partially formed from a water permeable material. A water permeable material is one that is configured to allow water to flow therethrough. It will be appreciated that a majority of the outer conduit wall 22 may be formed from the water permeable material. In the present arrangement, substantially all, e.g. an entirety, of the outer conduit wall 22 is formed from the water permeable material.

The water permeable material may also be configured to restrict flow of liquid water therethrough and/or is configured to restrict flow of gases therethrough. Forming the outer conduit wall from a material allowing water to flow therethrough and that allows, but restricts, the passage of liquid water and/or gases therethrough has been found to reduce the build-up of water and water vapour in the second flow passage. The restriction of the passage of liquid water and/or gases therethrough are lowered to within the clinical requirements of ventilator machine requirements, as defined in the standard ISO5367 for Anaesthetic and respiratory equipment - Breathing sets and connectors.

The outer conduit wall 22 is self-supporting. Put another way, the outer conduit 22 is sufficiently strong, or sufficiently study, so as to be self-supporting. In this way, no separate reinforcing members are required to be mounted/attached to the outer conduit 22 (which could be detrimental to the breathability of the outer conduit 22). This helps to increase the area of the outer conduit 22 that is water permeable. The outer conduit 22 defines an outer conduit wall having a thickness in the range 0.2mm to 1.0mm, optionally in the range 0.3mm to 0.9mm.

The outer conduit wall 22 includes a reinforcing arrangement. Put another way, the outer conduit wall 22 is arranged so as to define a reinforcing formation. This provides the outer conduit 22 with improved structural strength. The reinforcing arrangement is provided in the form of a corrugated region 38 of the outer conduit (i.e. the conduit wall of the outer conduit is corrugated). In the illustrated arrangement, the corrugated region extends over an entirety of the elongate length of the outer conduit 22. The corrugations in the outer conduit 22 work to increase the overall surface area of the water permeable outer conduit 22, thus increasing the area through which water within outer flow path can pass out of the breathing assembly 10.

The water permeable material of the outer conduit 22 is formed from an amphiphilic material. The water permeable material is formed from an amphiphilic block co-polymer. Put another way, the water permeable material is formed from a hydrophobic and hydrophilic block co-polymer. An example of one such material is a hydrophobic and hydrophilic poly (ethylene oxide) based block co-polymer. Further examples is suitable water permeable materials are: Nafion (RTM); Sympatex (RTM); Arnitel (RTM); Diaplex (RTM); and water permeable Hytrel (RTM), and it will be appreciated that any suitable water permeable material may be used.

In some arrangements, the water permeable material may be configured to absorb water vapour and liquid water. This has been found to further enhance the permeability of the outer conduit 22 to water vapour and liquid water, thereby further reducing the build-up of water and water vapour in the second flow passage.

In the present arrangement, the inner conduit wall 20 is insufficiently strong to be self-supporting. Put another way, the conduit wall of the inner conduit may have a low wall thickness (i.e. less than the outer conduit 22 wall thickness) such that the inner conduit 20 is insufficiently strong, or insufficiently study, so as to be self-supporting.

The inner conduit wall 20 is configured to prevent water and water vapour from flowing therethrough. The inner conduit wall 20 may be configured to prevent gases from flowing therethrough. As there is no transmission of moisture between the inspiratory and expiratory gases, the humidity of the inspiratory gases is able to be actively, rather than passively, controlled by the breathing assembly 10. In the present arrangement, the inner conduit wall 20 is formed from a thermoplastic elastomer. However, in alternative arrangements, it will be appreciated that any suitable material may be used such as thermoplastic extrudable polymers, for example polyethylene or polyurethane.

The inner conduit wall 20 is provided with a reinforcing arrangement. Incorporating a reinforcing arrangement on the inner conduit 20 enables the thickness of the wall of the inner conduit 20 to be reduced, thus reducing the amount of material used in the inner conduit 20. The reinforcing arrangement is provided in the form of a reinforcing rib 40. The reinforcing rib 40 extends around the inner conduit 20. In the illustrated arrangement, the reinforcing rib 40 extended helically (i.e. spirals) around the inner conduit 20.

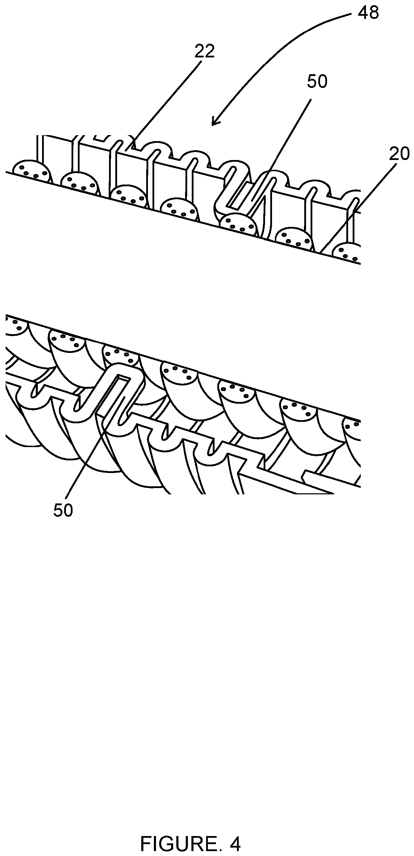

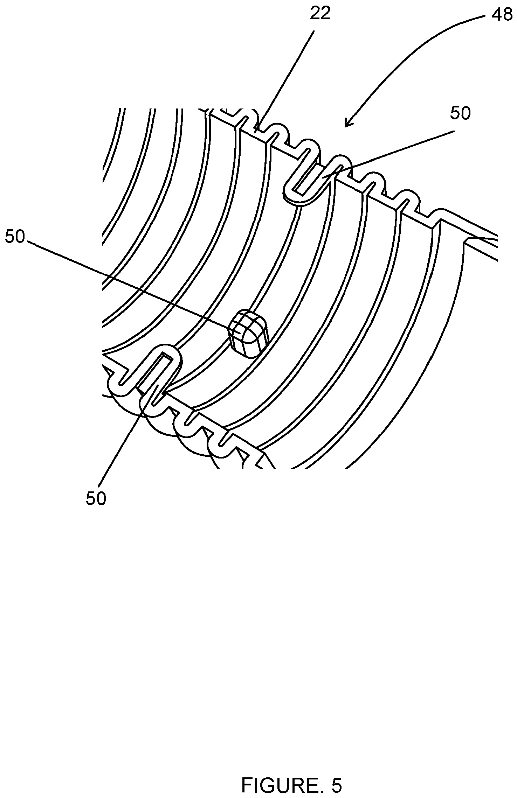

Referring now to Figures 4 and 5, the coaxial conduit assembly 18 includes a spacing arrangement between the inner conduit wall 20 and the outer conduit wall 22. The spacing arrangement includes a spacing assembly 48. The spacing arrangement may be provided with an array of spacing assemblies 48 along the elongate length of the coaxial conduit assembly 18.

Each spacing assembly 48 has a plurality of spacing members 50 positioned between the inner conduit 20 and the outer conduit 22. The plurality of spacing members 50 are equally spaced around the inner conduit 20. In the illustrated arrangement, four spacing members 50 are provided, but it will be appreciated that any suitable number of spacing members 50 may be provided such as three, five or more spacing members 50.

The spacing members 50 are configured and arranged to support the inner conduit 22. Put another way, the outer conduit 22 is configured to support the inner conduit 20. In the illustrated arrangement, the spacing members 50 project inwardly (i.e. radially inwardly) from the outer conduit wall 22. In alternative arrangements, the spacing members 50 may be separate from, but attached to, the inner conduit 20 and/or outer conduit 22.

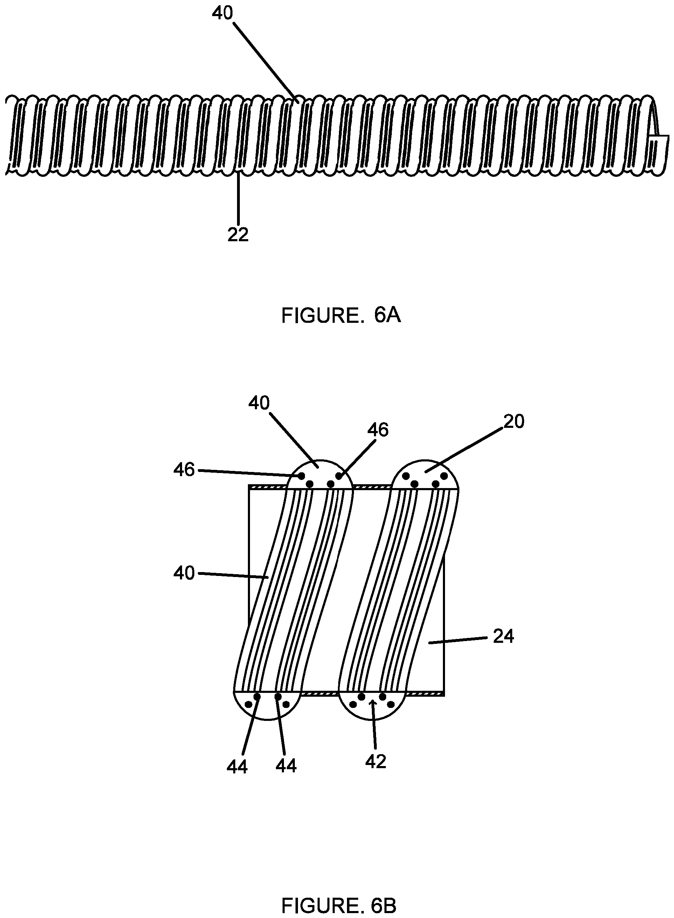

Referring now to Figures 6A and 6B, the breathing assembly 10 includes a heating arrangement for heating gases flowing along the coaxial conduit assembly 18.

The heating arrangement includes a first heating member 42. The first heating member 42 is configured and arranged to heat gases flowing along the first flow passage 24 and the second flow passage 26. The first heating member is configured to heat the inspiratory gases to a predetermined temperature of temperature range. The heating arrangement is configured such that the inspiratory gases maintain an absolute humidity of at least 33 mg/l. In the present arrangement, the first heating member is configured to heat the inspiratory gases to a temperature in the range 36°C to 41°C, for example in the range 37°C to 40°C. In the arrangement shown, the first heating member is configured to heat the inspiratory gases in the first flow passage to a temperature in the range 36°C to 41°C, for example in the range 37°C to 40°C. The first heating member comprises a heating power in the range 25 to 40 Watts, or in the range 27 to 35 Watts, optionally in the range 29 to 33 Watts, for example approximately 31 Watts.

The first heating member 42 is provided in the form of two spaced apart heating elements 44, e.g. wires. The first heating member 42 is embedded with the wall of the inner conduit wall 20. The first heating member 42 extends around, e.g. spirals around, the inner conduit wall of the inner conduit 20. In the illustrated arrangement, the first heating member 42 is embedded within the reinforcing rib 40. Put another way, the inner conduit 20 includes a reinforcing rib 40 having the first heating member 42 therein.

One or more electrical wires 46 are embedded in the wall of the inner conduit wall 20. The electrical wires 46 provide an electrical connection between the second temperature sensor 34 and the electrical connector 32. In the illustrated embodiment, two electrical wires 46 are embedded in the wall of the inner conduit 20. The electrical wire(s) 46 extend around, e.g. spiral around, the inner conduit wall of the inner conduit 20. In the illustrated arrangement, the one or more electrical wires 46 are embedded within the reinforcing rib 40. It will be appreciated in alternative arrangements that the one or more electrical wires 46 may be embedded in the wall of the inner conduit 20 but not within the reinforcing rib 40 of the inner conduit 20, or may be positioned within the second flow passage 26.

The heating arrangement may include a second heating member. The second heating member may be configured to heat gases flowing along the second flow passage 26. Provision of a dedicated heater for the second flow passage 26 enables the gases within the inner and outer flow passages 24, 26 to be heated to separate temperatures. The second heating member is configured to heat the outer conduit wall to a predetermined temperature range or a predetermined temperature. The water permeability of the material forming the outer conduit 22 will be dependent upon the temperature of said material. The provision of a heater configured to heat the wall of the outer conduit 22 enables the second heating member to heat the wall of the outer conduit 22 to a temperature or temperature range so as to maximise its permeability to water. It will be appreciated that the second heating element may either be positioned in the second flow passage 26 or may be embedded within a wall of the outer conduit 22. The second heating member comprises a heating power in the range 5 to 35 Watts.

Although not illustrated, it will be appreciated that the breathing assembly may be connected to a ventilator and a humidification chamber as a part of a breathing system. In such breathing systems, the humidification chamber will be arranged to be between the ventilator and the chamber end connector 14.

During inspiration, the ventilator (not shown) is vented through a limb/conduit to the humidification chamber. The inspiratory gases flowing through the humidification chamber are heated and humidified. The inspiratory gases then flow through the chamber end connector 14 and into the coaxial conduit assembly 18. The inspiratory gases are directed to flow along the inspiratory flow path (e.g. along the first flow passage 24 in the illustrated embodiment) and to the patient end connector 12. The inspiratory gases flow through the patient end connector 12 and are conveyed to a patient, e.g. via a catheter mount.

During expiration, expiratory gases are conveyed from the patient, e.g. via a catheter mount, to the patient end connector 12. The expiratory gases are then directed to flow along the expiratory flow path (e.g. along the second flow passage 26 in the illustrated embodiment) by the ventilator valve sequence inhibiting flow in the inspiratory path, but also optionally by a flow valve in the patient end connector. Expiratory gases then flow along the coaxial conduit assembly 18 and flow out of the breathing assembly 10 through the expiratory port 28, releasing the expiratory gasses to the atmosphere.

Although the teachings have been described above with reference to one or more preferred embodiments, it will be appreciated that various changes or modifications may be made without departing from the scope as defined in the appended claims.

## Claims

1. A breathing assembly (10) for supplying gases to a patient, the breathing assembly comprising:
a patient end connector (12) configured to be connectable to an airway device;
a chamber end connector (14) configured to be connectable to a humidification chamber and comprising an inspiratory inlet for receiving humidified inspiratory gases from the humidification chamber; and
a coaxial conduit assembly (18) extending between the patient end connector (12) and the chamber end connector (14) and comprising inner and outer conduits defined by inner and outer conduit walls, respectively, the inner conduit wall (20) arranged within the outer conduit wall (22) so as to define a first flow passage along the coaxial conduit assembly within the inner conduit and a second flow passage along the coaxial conduit assembly between the inner conduit and outer conduit,
wherein the breathing assembly is configured such that inspiratory gases are conveyed along the first flow passage and expiratory gases are conveyed along the second flow passage, in use, and
wherein the outer conduit wall (22) is at least partially formed from a water permeable material configured to allow water to flow therethrough, and **characterised in that** the breathing assembly comprises a heating arrangement configured and arranged to heat gases flowing along the coaxial conduit assembly;
wherein the heating arrangement comprises a first heating member (42) embedded within the inner conduit wall (20) of the inner conduit, the first heating member configured to heat gases flowing along the first flow passage; and
wherein the heating arrangement comprises a second heating member configured to heat gases flowing along the second flow passage, wherein the second heating element is either positioned in the second flow passage or is embedded within the outer conduit wall.

2. The breathing assembly according to claim 1, wherein the water permeable material is configured to allow flow of liquid water therethrough and configured to restrict flow of respiratory gases therethrough.

3. The breathing assembly according to claim 1 or claim 2, wherein substantially all, e.g. an entirety, of the outer conduit wall is formed from the water permeable material.

4. The breathing assembly according to any preceding claim, wherein the outer conduit wall (22) is self-supporting, optionally wherein the outer conduit comprises a reinforcing arrangement.

5. The breathing assembly according to any preceding claim, wherein the outer conduit wall (22) comprises a corrugated region (38), optionally wherein the corrugated region extends over an entirety of the elongate length of the outer conduit wall.

6. The breathing assembly according to any preceding claim, wherein the coaxial conduit assembly (18) comprises a spacing arrangement between the inner conduit wall (20) and the outer conduit wall (22), and wherein:
(i) the spacing arrangement comprises a plurality of spacing members (50) projecting inwardly from the outer conduit wall; or
(ii) wherein the spacing arrangement comprises at least one spacing assembly (48) provided along the elongate length of the coaxial conduit assembly (18), and wherein the or each spacing assembly comprises a plurality of spacing members (50) positioned between the inner conduit and the outer conduit.

7. The breathing assembly according to claim 6, wherein (ii) applies, and wherein the plurality of spacing members (50) are equally spaced around the inner conduit.

8. The breathing assembly according to any preceding claim, wherein the water permeable material is formed from an amphiphilic material.

9. The breathing assembly according to claim 8, wherein the water permeable material is formed from a hydrophobic and hydrophilic block co-polymer, optionally a hydrophobic and hydrophilic poly (ethylene oxide) based block co-polymer.

10. The breathing assembly according to any preceding claim, wherein the outer conduit wall (22) is configured to absorb water vapour and liquid water.

11. The breathing assembly according to any preceding claim, wherein the inner conduit wall (20) is configured to prevent water and water vapour from flowing therethrough, optionally wherein the inner conduit wall is configured to prevent gases from flowing therethrough.

12. The breathing assembly according to any preceding claim, wherein the inner conduit comprises a reinforcing arrangement (40), optionally wherein the reinforcing arrangement comprises a reinforcing rib extending around, e.g. helically around, the inner conduit wall (20).

13. The breathing assembly according to any preceding claim, wherein the inner conduit wall (20) comprises a reinforcing rib (40) extending therearound, and wherein the first heating member (42) is embedded within the reinforcing rib, optionally wherein the first heating member (42) extends around, e.g. spirals around, the inner conduit wall (20).

14. The breathing assembly according to claim 13, wherein the first heating member (42) is configured and arranged to heat inspiratory gases flowing along the first flow passage and to heat expiratory gases flowing along the second flow passage.

15. The breathing assembly according to any preceding claim, comprising:
(i) a catheter mount connected to the patient end connector; and/or
(ii) an expiratory port (28) for conveying expiratory gasses out of the breathing assembly, wherein the expiratory port is provided at or proximate to the patient end connector (12).

## Patentansprüche

1. Beatmungsanordnung (10) zum Zuführen von Gasen zu einem Patienten, wobei die Beatmungsanordnung Folgendes umfasst:
ein Patientenendverbindungsstück (12), das dazu ausgelegt ist, mit einer Atemwegsvorrichtung verbindbar zu sein;
ein Kammerendverbindungsstück (14), das dazu ausgelegt ist, mit einer Befeuchtungskammer verbindbar zu sein, und einen Inspirationseinlass zum Aufnehmen von befeuchteten Inspirationsgasen aus der Befeuchtungskammer umfasst; und
eine Koaxialleitungsanordnung (18), die sich zwischen dem Patientenendverbindungsstück (12) und dem Kammerendverbindungsstück (14) erstreckt und eine Innen- und eine Außenleitung umfasst, welche jeweils durch eine Innen- bzw. Außenleitungswand definiert sind, wobei die Innenleitungswand (20) innerhalb der Außenleitungswand (22) angeordnet ist, sodass ein erster Strömungsdurchgang entlang der Koaxialleitungsanordnung innerhalb der Innenleitung und ein zweiter Strömungsdurchgang entlang der Koaxialleitungsanordnung zwischen der Innenleitung und der Außenleitung definiert ist,
wobei die Beatmungsanordnung derart ausgelegt ist, dass bei Verwendung Inspirationsgase entlang des ersten Strömungsdurchgangs und Expirationsgase entlang des zweiten Strömungsdurchgangs befördert werden, und
wobei die Außenleitungswand (22) zumindest teilweise aus einem wasserdurchlässigen Material ausgebildet ist, welches dazu ausgelegt ist, Wasser hindurchströmen zu lassen, und **dadurch gekennzeichnet, dass** die Beatmungsanordnung eine Heizanordnung umfasst, die dazu ausgelegt und angeordnet ist, um Gase, die entlang der Koaxialleitungsanordnung strömen, zu erhitzen;
wobei die Heizanordnung ein erstes Heizelement (42), welches in der Innenleitungswand (20) der Innenleitung eingebettet ist, umfasst, wobei das erste Heizelement dazu ausgelegt ist, Gase, die entlang der Koaxialleitungsanordnung strömen, zu erhitzen, und
wobei die Heizanordnung ein zweites Heizelement umfasst, das dazu ausgelegt ist, Gase, die entlang der Koaxialleitungsanordnung strömen, zu erhitzen, wobei das zweite Heizelement entweder im zweiten Strömungsdurchgang positioniert oder in der Außenleitungswand eingebettet ist.

2. Beatmungsanordnung nach Anspruch 1, wobei das wasserdurchlässige Material dazu ausgelegt ist, das Strömen von Flüssigkeit durch dieses hindurch zu ermöglichen, und dazu ausgelegt ist, das Strömen von Atemgasen durch dieses hindurch einzuschränken.

3. Beatmungsanordnung nach Anspruch 1 oder 2, wobei die Außenleitungswand im Wesentlichen vollständig, z. B. in ihrer Gesamtheit, aus dem wasserdurchlässigen Material ausgebildet ist.

4. Beatmungsanordnung nach einem der vorangegangenen Ansprüche, wobei die Außenleitungswand (22) selbsttragend ist, wobei die Außenleitung gegebenenfalls eine Verstärkungsanordnung umfasst.

5. Beatmungsanordnung nach einem der vorangegangenen Ansprüche, wobei die Außenleitungswand (22) einen gewellten Bereich (38) umfasst, wobei der gewellte Bereich sich gegebenenfalls über die Gesamtheit der Längslänge der Außenleitungswand erstreckt.

6. Beatmungsanordnung nach einem der vorangegangenen Ansprüche, wobei die Koaxialleitungsanordnung (18) eine Beabstandungsanordnung zwischen der Innenleitungswand (20) und der Außenleitungswand (22) umfasst und wobei:
(i) die Beabstandungsanordnung eine Vielzahl von Beabstandungselementen (50) umfasst, die von der Außenleitungswand nach innen wegstehen; oder
(ii) die Beabstandungsanordnung zumindest eine Beabstandungsanordnung (48), die entlang der Längslänge der Koaxialleitungsanordnung (18) bereitgestellt ist, umfasst, und wobei die oder jede Beabstandungsanordnung eine Vielzahl von Beabstandungselementen (50), die zwischen der Innenleitung und der Außenleitung positioniert sind, umfasst.

7. Beatmungsanordnung nach Anspruch 6, wobei (ii) zutrifft, und wobei die Vielzahl von Beabstandungselementen (50) gleichmäßig um die Innenleitung herum beabstandet sind.

8. Beatmungsanordnung nach einem der vorangegangenen Ansprüche, wobei das wasserdurchlässige Material aus einem amphiphilen Material ausgebildet ist.

9. Beatmungsanordnung nach Anspruch 8, wobei das wasserdurchlässige Material aus einem hydrophoben und hydrophilen Block-Co-Polymer, gegebenenfalls einem hydrophoben und hydrophilen Block-Co-Polymer auf Poly(ethylenoxid)-Basis, ausgebildet ist.

10. Beatmungsanordnung nach einem der vorangegangenen Ansprüche, wobei die Außenleitungswand (22) dazu ausgelegt ist, Wasserdampf und flüssiges Wasser zu absorbieren.

11. Beatmungsanordnung nach einem der vorangegangenen Ansprüche, wobei die Innenleitungswand (20) dazu ausgelegt ist, Wasser und Wasserdampf daran zu hindern, durch diese hindurchzuströmen, wobei die Innenleitungswand gegebenenfalls dazu ausgelegt ist, Gase daran zu hindern, durch diese hindurchzuströmen.

12. Beatmungsanordnung nach einem der vorangegangenen Ansprüche, wobei die Innenleitung eine Verstärkungsanordnung (40) umfasst, wobei die Verstärkungsanordnung gegebenenfalls eine Verstärkungsrippe umfasst, die sich, z. B. helixförmig, um die Innenleitungswand (20) herum erstreckt.

13. Beatmungsanordnung nach einem der vorangegangenen Ansprüche, wobei die Innenleitungswand (20) eine sich um sie herum erstreckende Verstärkungsrippe (40) umfasst und wobei das erste Heizelement (42) in der Verstärkungsrippe eingebettet ist, wobei sich das erste Heizelement (42) gegebenenfalls, z. B. spiralförmig, um die Innenleitungswand (20) herum erstreckt.

14. Beatmungsanordnung nach Anspruch 13, wobei das erste Heizelement (42) dazu ausgelegt und angeordnet ist, Inspirationsgase, die entlang des ersten Strömungsdurchgangs strömen, zu erhitzen und Expirationsgase, die entlang des zweiten Strömungsdurchgangs strömen, zu erhitzen.

15. Beatmungsanordnung nach einem der vorangegangenen Ansprüche, umfassend:
(i) eine Katheterhalterung, die mit dem Patientenendverbindungsstück verbunden ist; und/oder
(ii) einen Expirationsanschluss (28) zum Hinausbefördern von Expirationsgasen aus der Beatmungsanordnung, wobei der Expirationsanschluss auf dem oder in der Nähe des Patientenendverbindungsstücks (12) bereitgestellt ist.

## Revendications

1. Ensemble respiratoire (10) pour fournir des gaz à un patient, l'ensemble respiratoire comprenant :
un raccord d'extrémité de patient (12) configuré pour pouvoir être raccordé à un dispositif de voies aériennes ;
un raccord d'extrémité de chambre (14) configuré pour pouvoir être raccordé à une chambre d'humidification et comprenant une entrée d'inspiration pour recevoir des gaz d'inspiration humidifiés provenant de la chambre d'humidification ; et
un ensemble de conduits coaxiaux (18) s'étendant entre le raccord d'extrémité de patient (12) et le raccord d'extrémité de chambre (14) et comprenant des conduits interne et externe définis par des parois de conduit interne et externe, respectivement, la paroi de conduit interne (20) étant agencée à l'intérieur de la paroi de conduit externe (22) de manière à définir un premier passage d'écoulement le long de l'ensemble de conduits coaxiaux à l'intérieur du conduit interne et un second passage d'écoulement le long de l'ensemble de conduits coaxiaux entre le conduit interne et le conduit externe,
dans lequel l'ensemble respiratoire est configuré de telle sorte que des gaz d'inspiration sont transportés le long du premier passage d'écoulement et que des gaz d'expiration sont transportés le long du second passage d'écoulement, en utilisation, et
dans lequel la paroi de conduit externe (22) est au moins partiellement formée à partir d'un matériau perméable à l'eau configuré pour permettre à l'eau de s'écouler à travers celui-ci, et **caractérisé en ce que** l'ensemble respiratoire comprend un agencement de chauffage configuré et agencé pour chauffer des gaz s'écoulant le long de l'ensemble de conduits coaxiaux ;
dans lequel l'agencement de chauffage comprend un premier élément chauffant (42) intégré à l'intérieur de la paroi de conduit interne (20) du conduit interne, le premier élément chauffant étant configuré pour chauffer des gaz s'écoulant le long du premier passage d'écoulement ; et
dans lequel l'agencement de chauffage comprend un second élément chauffant configuré pour chauffer des gaz s'écoulant le long du second passage d'écoulement, dans lequel le second élément chauffant est soit positionné dans le second passage d'écoulement, soit est intégré à l'intérieur de la paroi de conduit externe.

2. Ensemble respiratoire selon la revendication 1, dans lequel le matériau perméable à l'eau est configuré pour permettre un écoulement d'eau liquide à travers celui-ci et configuré pour restreindre un écoulement de gaz respiratoires à travers celui-ci.

3. Ensemble respiratoire selon la revendication 1 ou la revendication 2, dans lequel sensiblement la totalité, par exemple la totalité, de la paroi de conduit externe est formée à partir du matériau perméable à l'eau.

4. Ensemble respiratoire selon l'une quelconque des revendications précédentes, dans lequel la paroi de conduit externe (22) est autoportante, facultativement dans lequel le conduit externe comprend un agencement de renforcement.

5. Ensemble respiratoire selon l'une quelconque des revendications précédentes, dans lequel la paroi de conduit externe (22) comprend une région ondulée (38), facultativement dans lequel la région ondulée s'étend sur une totalité de la longueur allongée de la paroi de conduit externe.

6. Ensemble respiratoire selon l'une quelconque des revendications précédentes, dans lequel l'ensemble de conduits coaxiaux (18) comprend un agencement d'espacement entre la paroi de conduit interne (20) et la paroi de conduit externe (22), et dans lequel :
(i) l'agencement d'espacement comprend une pluralité d'éléments d'espacement (50) faisant saillie vers l'intérieur à partir de la paroi de conduit externe ; ou
(ii) dans lequel l'agencement d'espacement comprend au moins un ensemble d'espacement (48) prévu le long de la longueur allongée de l'ensemble de conduits coaxiaux (18), et dans lequel le ou chaque ensemble d'espacement comprend une pluralité d'éléments d'espacement (50) positionnés entre le conduit interne et le conduit externe.

7. Ensemble respiratoire selon la revendication 6, dans lequel (ii) s'applique, et dans lequel la pluralité d'éléments d'espacement (50) sont espacés de manière égale autour du conduit interne.

8. Ensemble respiratoire selon l'une quelconque des revendications précédentes, dans lequel le matériau perméable à l'eau est formé à partir d'un matériau amphiphile.

9. Ensemble respiratoire selon la revendication 8, dans lequel le matériau perméable à l'eau est formé à partir d'un copolymère séquencé hydrophobe et hydrophile, facultativement un copolymère séquencé à base de poly (oxyde d'éthylène) hydrophobe et hydrophile.

10. Ensemble respiratoire selon l'une quelconque des revendications précédentes, dans lequel la paroi de conduit externe (22) est configurée pour absorber de la vapeur d'eau et de l'eau liquide.

11. Ensemble respiratoire selon l'une quelconque des revendications précédentes, dans lequel la paroi de conduit interne (20) est configurée pour empêcher de l'eau et de la vapeur d'eau de s'écouler à travers elle, facultativement dans lequel la paroi de conduit interne est configurée pour empêcher des gaz de s'écouler à travers elle.

12. Ensemble respiratoire selon l'une quelconque des revendications précédentes, dans lequel le conduit interne comprend un agencement de renforcement (40), facultativement dans lequel l'agencement de renforcement comprend une nervure de renforcement s'étendant, par exemple de manière hélicoïdale, autour de la paroi de conduit interne (20).

13. Ensemble respiratoire selon l'une quelconque des revendications précédentes, dans lequel la paroi de conduit interne (20) comprend une nervure de renforcement (40) s'étendant autour de celle-ci, et dans lequel le premier élément chauffant (42) est intégré à l'intérieur de la nervure de renforcement, facultativement dans lequel le premier élément chauffant (42) s'étend, par exemple en spirale, autour de la paroi de conduit interne (20).

14. Ensemble respiratoire selon la revendication 13, dans lequel le premier élément chauffant (42) est configuré et agencé pour chauffer des gaz d'inspiration s'écoulant le long du premier passage d'écoulement et pour chauffer des gaz d'expiration s'écoulant le long du second passage d'écoulement.

15. Ensemble respiratoire selon l'une quelconque des revendications précédentes, comprenant :
(i) un support de cathéter raccordé au raccord d'extrémité de patient ; et/ou
(ii) un orifice d'expiration (28) pour transporter des gaz d'expiration hors de l'ensemble respiratoire, dans lequel l'orifice d'expiration est prévu au niveau ou à proximité du raccord d'extrémité de patient (12).
